(19) Europäisches Patentamt
European Patent Office
Office européen des brevets

(11) Veröffentlichungsnummer: **0 263 299**
**B1**

(12) **EUROPÄISCHE PATENTSCHRIFT**

(45) Veröffentlichungstag der Patentschrift: **07.11.90**

(21) Anmeldenummer: **87112651.2**

(22) Anmeldetag: **31.08.87**

(51) Int. Cl.[5]: **C07D 413/04,** C07D 487/04, C07D 471/04, C07F 7/08, C07D 265/36, A01N 43/84, A01N 43/90 // (C07D487/04, 249:00, 237:00),(C07D471/04, 235:00, 221:00)

(54) **Neue Benzoxazine.**

(30) Priorität: **09.09.86 JP 210725/86**
**10.02.87 JP 27194/87**

(43) Veröffentlichungstag der Anmeldung:
**13.04.88 Patentblatt 88/15**

(45) Bekanntmachung des Hinweises auf die Patenterteilung:
**07.11.90 Patentblatt 90/45**

(84) Benannte Vertragsstaaten:
**BE CH DE FR GB IT LI NL**

(56) Entgegenhaltungen:
**EP-A- 0 170 191**
**EP-A- 0 176 101**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., Itohpia Nihonbashi Honcho Building 7-1, Nihonbashi Honcho 2-chome, Chuo-ku Tokyo 103(JP)**

(72) Erfinder: **Kume, Toyohiko, 6-7-8 Asahigaoka, Hino-Shi Tokyo(JP)**
Erfinder: **Goto, Toshio, 3454-21, Honmachida, Machida-shi Tokyo(JP)**
Erfinder: **Kamochi, Atsumi, 2-24-10, Higashi-Toyoda, Hino-shi Tokyo(JP)**
Erfinder: **Yamaguchi, Naoko, 4-6-7, Shinmei, Hino-shi Tokyo(JP)**
Erfinder: **Yanagi, Akihiko, 2-1200-1 Nagabuchi, Oume-shi Tokyo(JP)**
Erfinder: **Hayakawa, Hidenori, 1631-13, Shimokuzawa, Sagamihara-shi Kanagawa-ken(JP)**
Erfinder: **Yagi, Shigeki, 1-30-7, Izumi, Suginami-ku Tokyo(JP)**
Erfinder: **Miyauchi, Hiroshi, 39-15, Namiki-cho, Hachioji-shi, Tokyo(JP)**

(74) Vertreter: **Ernst, Hilmar, Dr. et al, Bayer AG Konzernverwaltung RP Patentabteilung, D-5090 Leverkusen 1, Bayerwerk(DE)**

**EP 0 263 299 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benzoxazine, Verfahren zu ihrer Herstellung und ihre Verwendung als Herbizide.

Es wurde bereits offenbart, daß bestimmte Tetrahydrophthalimide herbizide Aktivitäten besitzen (siehe die JP-OS 30 586/1986).

Nunmehr wurden neue Benzoxazine der Formel (I)

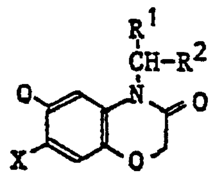

(I)

gefunden, in der
X Wasserstoff oder Halogen ist,
$R^1$ Wasserstoff oder $C_1$-$C_2$-Alkyl ist,
$R^2$ Cyano, Trimethylsilyl, Trimethylsilylmethoxycarbonyl, $C_1$-$C_4$-Alkylthio oder Cyclopropyl ist und
Q eine der folgenden Gruppen ist

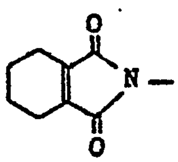
,
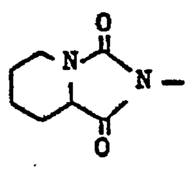
,
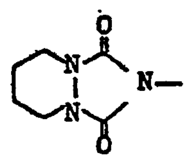
oder
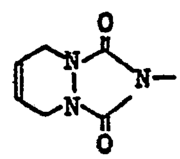

Die Verbindungen der Formel (I) werden mittels eines Verfahrens erhalten, bei dem

a) Verbindungen der Formel (II)

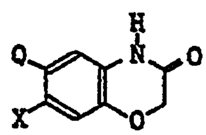

(II)

in der X and Q die oben angegebenen Bedeutungen haben, mit Verbindungen der Formel (III)

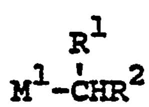

(III)

in der $R^1$ und $R^2$ die oben angegebenen Bedeutungen haben und $M^1$ Halogen ist, in Gegenwart eines inerten Lösungsmittels, gegebenenfalls in Gegenwart einer Base, umgesetzt werden oder

b) in dem Fall, in dem Q die folgende Gruppe ist

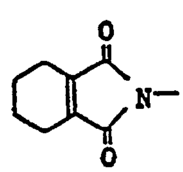

Verbindungen der Formel (IV)

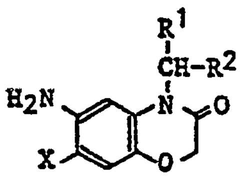

(IV)

in der X, $R^1$ und $R^2$ die oben angegebenen Bedeutungen haben, mit Tetrahydrophthalsäureanhydrid der Formel

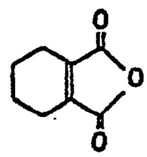

in Gegenwart von Essigsäure umgesetzt werden oder

c) in dem Fall, in dem Q eine der folgenden Gruppen ist

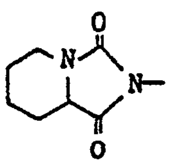 , 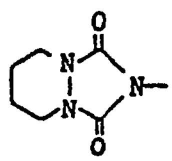 oder 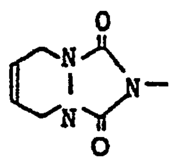

Verbindungen der Formel (V)

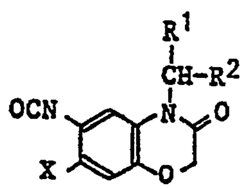

(V)

in der X, $R^1$ und $R^2$ die oben angegebenen Bedeutungen haben, mit Verbindungen der Formel (VI)

W - C O O T(VI),

in der W eine der folgenden Gruppen ist

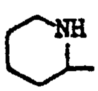 , 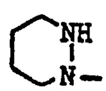 oder 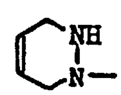

und

T Wasserstoff oder Alkyl ist,
in Gegenwart eines inerten Lösungsmittels umgesetzt werden und dann die genannten Produkte in Gegenwart einer Säure oder einer Base cyclisiert werden.

Die neuen Benzoxazine zeigen sehr gute herbizide Eigenschaften.

Überraschenderweise zeigen die erfindungsgemäßen Benzoxazine nicht nur eine wesentlich stärkere herbizide Wirkung als die aus dem oben genannten Stand der Technik bekannten, sondern auch eine günstige Verträglichkeit mit Nutzpflanzen, ohne Phytotoxizität.

Unter den Benzoxazinen gemäß der Erfindung sind bevorzugte Verbinduungen der Formel (I) diejenigen, in denen

X Wasserstoff, Fluor oder Chlor ist,
$R^1$ Wasserstoff oder Methyl ist,

$R^2$ Cyano, Trimethylsilyl, Trimethylsilylmethoxycarbonyl, Methylthio oder Cyclopropyl ist und
Q eine der folgenden Gruppen ist

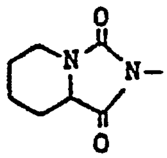 , 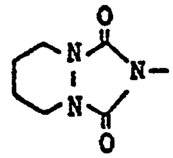 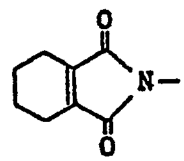 , oder 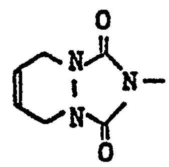

Ganz besonders bevorzugte Benzoxazine der Formel (I) sind diejenigen, in denen
X Wasserstoff oder Fluor ist,
$R^1$ Wasserstoff oder Methyl ist,
$R^2$ Cyano ist und
Q eine der folgenden Gruppen ist

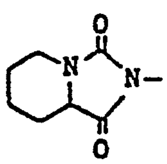 , 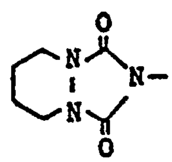 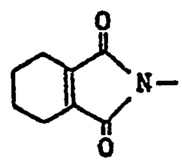 , oder 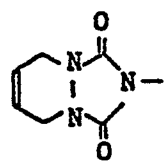

Speziell erwähnt seien die folgenden Verbindungen:

2-[4-Cyanomethyl-7-fluoro-2H-1,4-benzoxazin-3(4H)-on-6-yl]-4,5,6,7-tetrahydro-2H-isoindol-1,3-dion, 2-[4-(1-Cyanoethyl)-7-fluoro-2H-1,4-benzoxazin-3(4H)-on-6-yl]-4,5,6,7-tetrahydro-2H-isoindol-1,3-dion, 3-[4-Cyanomethyl-7-fluoro-2H-1,4-benzoxazin-3(4H)-on-6-yl]-1,5-tetramethylen-1,3-diazolidin-2,4-dion und 2-[4-Cyanomethyl-7-fluoro-2H-1,4-benzoxazin-3(4H)-on-6-yl]-hexahydro-1H-(1,2,4) triazolo(1,2-a)pyridazin-1,3-dion.

Wenn beispielsweise in dem vorstehenden Verfahren a) 2-[7-Fluoro-2H-1,4-benzoxazin-3(4H)-on-6-yl]-4,5,6,7-tetrahydro-2H-isoindol-1,3-dion und Chloroacetonitril als Ausgangsstoffe eingesetzt werden, kann der Reaktionsverlauf durch die folgende Gleichung dargestellt werden:

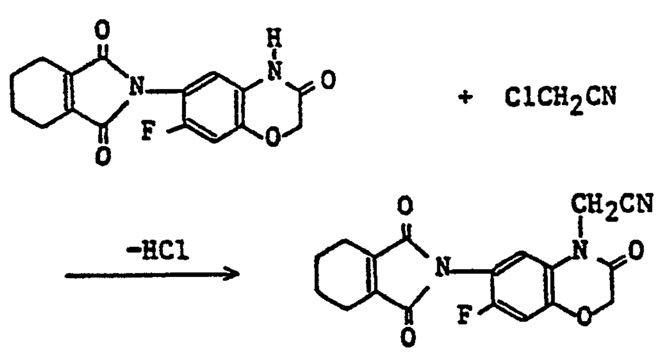


Wenn beispielsweise in dem vorstehenden Verfahren b) 6-Amino-4-(1-Cyanoethyl)-2H-1,4-benzoxazin-3(4H)-on und Tetrahydrophthalsäureanhydrid als Ausgangsstoffe eingesetzt werden, kann der Reaktionsverlauf durch die folgende Gleichung dargestellt werden:

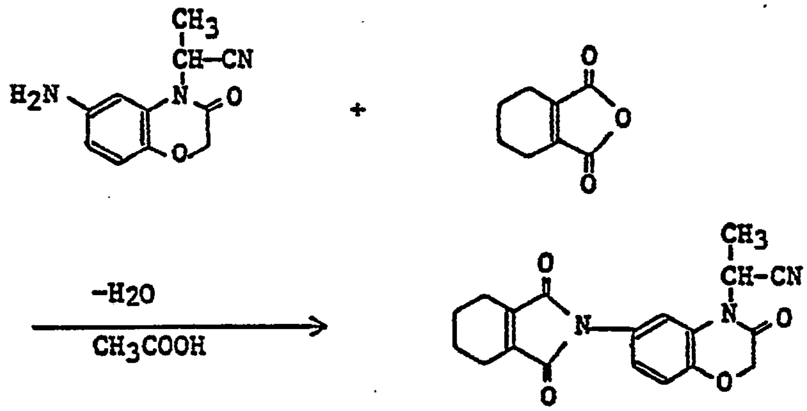


Wenn beispielsweise in dem vorstehenden Verfahren c) 4-Cyanomethyl-6-isocyanato-7-fluoro-2H-1,4-benzoxazin-3(4H)-on und 1-Ethoxycarbonyl-hexahydropyridazin als Ausgangsstoffe eingesetzt werden und dann das Produkt cyclisiert wird, kann der Reaktionsverlauf durch die folgende Gleichung dargestellt werden:

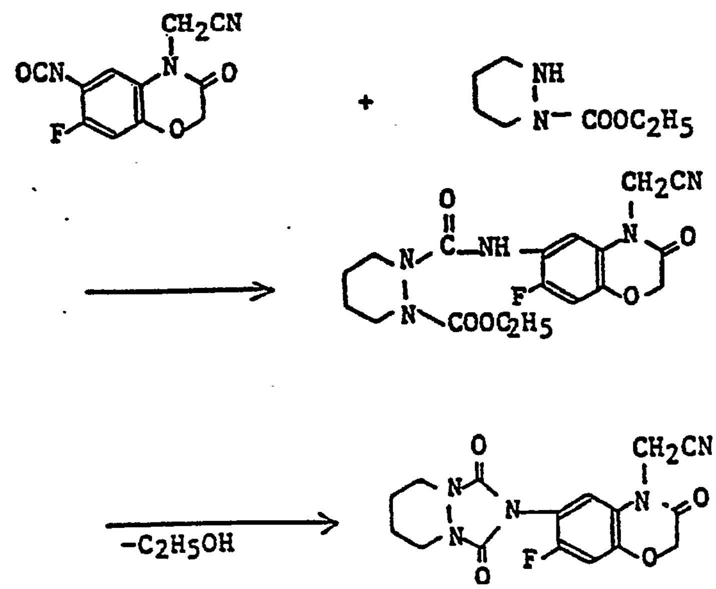


In dem Verfahren a) bezeichnen die Verbindungen der Formel (II) als Ausgangsstoffe Verbindungen auf der Basis der oben gegebenen Definitionen für X und Q.

In der Formel (II) haben X und Q vorzugsweise die bereits im Vorstehenden angegebenen Bedeutungen.

Die Verbindungen der Formel (II) sind bereits bekannt und können allgemein mittels eines Verfahrens erhalten werden, das in Agr. Biol. Chem. 40, S. 745-751 (1976) oder in Pestic. Biochem. Physiol. 14, S. 153-160, beschrieben ist, wie es im Folgenden gezeigt wird:

In dem Fall in dem Q die folgende Gruppe ist

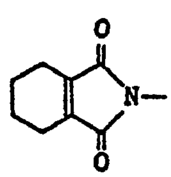

können die Verbindungen der Formel (II) erhalten werden, wenn Verbindungen der Formel (VII)

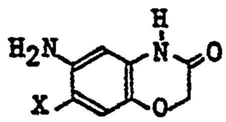

(VII)

in der X die oben angegebenen Bedeutungen hat, mit Tetrahydrophthalsäureanhydrid in Essigsäure unter Rückfluß ummgesetzt werden.

Die Verbindungen der vorstehenden Formel (VII) können im allgemeinen erhalten werden, wenn Verbindungen der Formel (VIII)

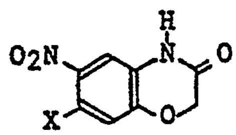

(VIII)

in der X die oben angegebenen Bedeutungen hat, reduziert werden.

Zum Beispiel kann in dem Fall, in dem X Wasserstoff ist, die betreiffende Verbindung der Formel (VII) erhalten werden, wenn 6-Nitro-1,4-benzoxazin-3(4H)-on reduziert wird, und 6-Nitro-1,4-benzoxazin-3(4H)-on kann erhalten werden, wenn das bekannte 2-Hydroxy-5-nitroanilin mit dem Chloroacetylchlorid nach einem in Synthesis 1984, S. 851, oder in der JP-OS 125 529/1974 beschrieben Verfahren umgesetzt wird.

In dem Fall, in dem X Fluor ist, kann die betreffende Verbindung der Formel (VII) erhalten werden, wenn 7-Fluoro-6-nitro-1,4-benzoxazin-3(4H)-on in der gleichen Weise wie oben erwähnt reduziert wird, und 7-Fluoro-6-nitro-1,4-benzoxazin-3(4H)-on kann erhalten werden, wenn 7-Fluoro-1,4-benzoxazin-3(4H)-on nach einem in Synthesis Organic Chemistry, R.B. Wagner und H. D. Zook, erschienen bei John Wiley & Sons Inc., 1953, S. 746, nitriert wird. Das 7-Fluoro-1,4-benzoxazin-3(4H)-on kann erhalten werden, wenn 4-Fluoro-2-hydroxyanilin mit Chloroacetylchlorid umgesetzt wird, und 4-Fluoro-2-hydroxyanilin schließlich kann erhalten werden, wenn das bekannte 5-Fluoro-2-nitrophenol entsprechend den Angaben im Vorstehenden reduziert wird.

In dem Fall, in dem Q eine der folgenden Gruppen ist

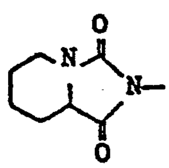 , 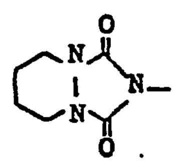 . oder 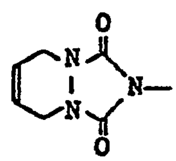 ,

können die Verbindungen der Formel (II) erhalten werden, wenn Verbindungen der Formel (IX)

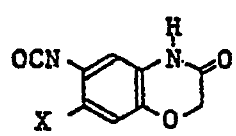

(IX)

in der X die oben angegebenen Bedeutungen hat, mit den oben bezeichneten Verbindungen der Formel (VI) umgesetzt werden und die dabei erhaltenen Produkte in Gegenwart einer Base cyclisiert werden.

Die Verbindungen der Formel (IX) können erhalten werden, wenn die oben bezeichneten Verbindungen der Formel (VII) mit Phosgen oder mit Trichloromethylchlorameisensäureester umgesetzt werden.

Als Beispiele für die Verbindungen der Formel (II) seien erwähnt:

2-[7-Fluoro-2H-1,4-benzoxazin-3(4H)-on-6yl]-4,5,6,7-t trahydro-2H-isoindol-1,3-dion,
2-[7-Fluoro-2H-1,4-b enzoxazin-3(4H)-on-6yl]-hexahydro1H-(1,2,4)triazolo(1,2-a)pyridazin-1,3 dion und
2-[7-Fluoro-2H-1,4-benzoxazin-3(4H)-on-6yl]-5,6,7,8-tetrahydroimidazo(1,5-a)pyridin-1,3(2H,8aH)-dion.

In dem Verfahren a) bezeichnen die Verbindungen der Formel (III), die ebenfalls Ausgangsstoffe sind, Verbindungen auf der Basis der oben gegebenen Definitionen für $R^1$, $R^2$ und $M^1$.

In der Formel (III) haben $R^1$ und $R^2$ vorzugsweise die bereits im Vorstehenden angegebenen Bedeutungen, und $M^1$ bezeichnet vorzugsweise Chlor oder Brom.

Die Verbindungen der Formel (III) sind bereits bekannt. Als Beispiele seien erwähnt:

Chloroacetonitril,
Trimethylsilylmethylbromoacetat und
Trimethylsilylmethylchloroacetat.

Beispielsweise kann Trimethylsilylmethylbromoacetat erhalten werden, wenn Trimethylsilylmethanol mit Bromoacetylchlorid oder Bromoacetylbromid in Gegenwart einer Base wie Treiethylamin umgesetzt wird.

In dem Verfahren b) bezeichnen die Verbindungen der Formel (IV) als Ausgangsstoffe Verbindungen auf der Basis der oben gegebenen Definitionen für X, $R^1$ und $R^2$.

In der Formel (IV) haben X, $R^1$ und $R^2$ vorzugsweise die bereits im Vorstehenden angegebenen Bedeutungen.

Die Verbindungen der Formel (IV) sind neu und können in einfacher Weise erhalten werden, wenn Verbindungen der Formel (X)

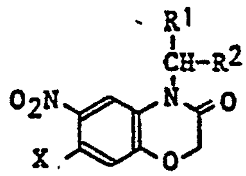

(X)

in der X, $R^1$ und $R^2$ die im Vorstehenden angegebenen Bedeutungen haben, nach einem in J. Am. Chem. Soc. 77, S. 6266, beschriebenen Verfahren reduziert werden.

Die Verbindungen der Formel (X) sind neu und können erhalten werden, wenn Verbindungen der Formel (XI)

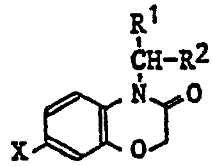

(XI)

in der X, $R^1$ und $R^2$ die im Vorstehenden angegebenen Bedeutungen haben, nitriert werden oder die oben bezeichneten Verbindungen der Formel (III) mit den oben bezeichneten Verbindungen der Formel (VIII) umgesetzt werden.

Die Verbindungen der Formel (XI) sind ebenfalls neu und können erhalten werden, wenn Verbindungen der Formel (XII)

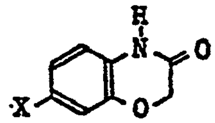

(XII)

in der X die im Vorstehenden angegebenen Bedeutungen hat, mit den oben bezeichneten Verbindungen der Formel (III) umgesetzt werden.

Die Verbindungen der Formel (XII) können erhalten werden, wenn Verbindungen der Formel (XIII)

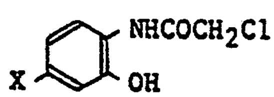

(XIII)

in der X die im Vorstehenden angegebenen Bedeutungen hat, in Gegenwart einer Base cyclisiert werden.

Die Verbindungen der Formel (XIII) können erhalten werden, wenn Verbindungen der Formel (XIV)

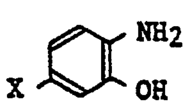

(XIV) ,

in der X die im Vorstehenden angegebenen Bedeutungen hat, nach einem herkömmlichen Verfahren mit Chloroacetylchlorid umgesetzt werden.

Die Verbindung der Formel (XIV) entspricht in dem Fall, in dem X Fluor ist, dem bereits im Vorstehenden genannten 4-Fluoro-2-hydroxyanilin. In dem Fall, in dem X Chlor ist, kann die Verbindung der Formel (XIV) erhalten werden, wenn das bekannte 6-Chloro-2(3H)-benzoxazolon nach einem in J. Am. Chem. Soc. 71, S. 1265-1268, beschriebenen Verfahren hydrolysiert wird.

In dem Fall in dem $R^2$ Alkylthio ist, wird $R^2$ durch $R^3$ ersetzt, und die Verbindungen der Formel (X) können erhalten werden, wenn Verbindungen der Formel (XV)

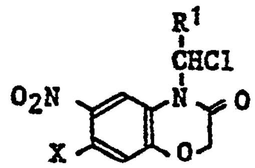

(XV)

in der X und $R^1$ die im Vorstehenden angegebenen Bedeutungen haben, mit Verbindungen der Formel (XVI)

$R^3$ - $M^2$ (XVI)

umgesetzt werden, in der
$R^3$ $C_{1-4}$-Alkylthio ist und
$M^2$ ein Alkalimetall ist.

Die Verbindungen der Formel (XV) können erhalten werden, wenn die oben erwähnten Verbindungen der Formel (VIII) mit Aldehyden der Formel

$R^1$ - CHO (XVII),

in der $R^1$ die im Vorstehenden angegebenen Bedeutungen hat, zu Verbindungen der Formel (XVIII)

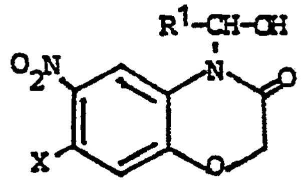

in der X und $R^1$ die im Vorstehenden angegebenen Bedeutungen haben, umgesetzt werden und diese Produkte mit Thionylchlorid umgesetzt werden.

In dem Verfahren c) bezeichnen die Verbindungen derFormel (V) als Ausgangsstoffe Verbindungen auf der Basis der oben gegebenen Definitionen für X, $R^1$ und $R^2$.

In der Formel (V) haben X, $R^1$ und $R^2$ vorzugsweise die bereits im Vorstehenden angegebenen Bedeutungen.

Die Verbindungen der Formel (V) können erhalten werden, wenn die oben bezeichneten Verbindungen der Formel (IV) mit Trichloromethylchlorameisensäureester oder Phosgen umgesetzt werden.

Die Verbindungen der Formel (VI), die ebenfalls Ausgansstoffe sind, bezeichnen Verbindungen auf der Basis der oben gegebenen Definitionen für W und T.

Die Verbindungen der Formel (VI) entsprechen in dem Fall, in dem W die folgende Gruppe ist,

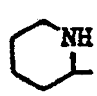

der Pipercolinsäure oder ihrem Ethylester, die im Handel erhältlich sind, und in dem Fall, in dem W eine der folgenden Gruppen ist,

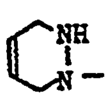 oder 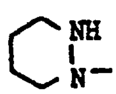

können die entsprechenden angestrebten Verbindungen der Formel (VI), 1-Ethoxycarbonyl-1,2,3,6-tetrahydropyridazin oder 1-Ethoxycarbonylhexahydropyridazin, auf dem Wege über 1,2-Bis(ethoxycarbonyl)-1,2,3,6-Tetrahydropyridazin, das durch Umsetzung von 1,3-Butadien und Diethylazodicarboxylat als den Ausgangsstoffen hergestellt wurde, erhalten werden.

Die Verbindungen der Formeln (IV), (X), (XI), (XV) und (XVIII) sind neu und lassen sich summarisch in der folgenden Formel (XIX) zusammenfassen

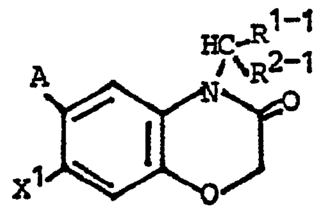

(XIX)

in der
$R^{1-1}$ Wasserstoff, Methyl oder Ethyl ist,
$R^{2-1}$ Cyano, Hydroxy, Chlor, Trimethylsilyl, Trimethylsilylmethoxycarbonyl, $C_1$-$C_4$-Alkylthio oder Cyclopropyl ist,
A Wasserstoff, Amino oder Nitro ist und
$X^1$ Wasserstoff, oder Halogen ist,
mit der Maßgabe, daß wenn A nitro ist, $R^{2-1}$ Chlor oder Hydroxy ist.

Bevorzugte Verbindungen der Formel (XIX) sind diejenigen, in denen
$R^{1-1}$ Wasserstoff oder Methyl ist,
$R^{2-1}$ Cyano, Hydroxy, Chlor oder $C_1$-$C_4$-Alkylthio ist,
A Wasserstoff, Amino oder Nitro ist und
$X^1$ Wasserstoff, Fluor oder Chlor ist,
mit der Maßgabe, daß, wenn A Nitro ist, $R^{2-1}$ Chlor oder Hydroxy ist.

Besonders bevorzugte Verbindungen der Formel (XIX) sind diejenigen, in denen
$R^{1-1}$ Wasserstoff oder Methyl ist,
$R^{2-1}$ Cyano, Hydroxy, Chlor oder Methylthio ist,
A Wasserstoff oder Nitro ist und
$X^1$ Wasserstoff oder Fluor ist,
mit der Maßgabe, daß, wenn A Nitro ist, $R^{2-1}$ Chlor oder Hydroxy ist.

Geeignete Verdünnungsmittel bei dem Verfahren a) sind sämtliche inerten organischen Lösungsmittel.

Beispiele für diese umfassen vorzugsweise Wasser, Nitrile wie Acetonitril, Alkohole wie Ethanol, Säureamide wie Dimethylformamid, Sulfoxide wie Dimethylsulfoxid, Ketone wie Aceton, und dergleichen.

Das Verfahren a) kann auch in Gegenwart einer Base durchgeführt werden. Beispiele für die Basen umfassen vorzugsweise Natriumcarbonat, Natriumhydrid, Kaliumcarbonat, Kaliumhydroxid, Natriumhydroxid, Natriummethoxid, Natriumethoxid, Kalium-t-butoxid und dergleichen.

Die Reaktionstemperaturen des Verfahrens a) können innerhalb eines Bereichs von beträchtlicher Breite variiert werden. Im allgemeinen wird die Reaktion bei einer Temperatur zwischen etwa 20 °C und etwa 150 °C, vorzugsweise zwischen etwa 30 °C und etwa 100 °C, durchgeführt.

Die Reaktion des Verfahrens a) kann unter normalem, erhöhtem oder vermindertem Druck durchgeführt werden.

Bei der Durchführung des Verfahrens a) können beispielsweise etwa 1 bis 1,2 mol der Verbindungen der Formel (III) auf 1 mol der Verbindungen der Formel (II) eingesetzt werden, und diese Verbindungen werden miteinander in Gegenwart eines inerten Lösungsmittels und in Gegenwart einer Base umgesetzt, so daß die angestrebten Verbindungen der Formel (I) erhalten werden können.

Bei der Durchführung des Verfahrens b) umfassen geeignete Verdünnungsmittel die gleichen Lösungsmittel, die beispielhaft für das Verfahren a) genannt sind.

Die Reaktionstemperaturen des Verfahrens b) können innerhalb eines Bereichs von beträchtlicher Breite variiert werden. Im allgemeinen wird die Reaktion bei einer Temperatur zwischen etwa 70 °C und etwa 280 °C, vorzugsweise zwischen etwa 80 °C und etwa 140 °C, durchgeführt.

Die Reaktion des Verfahrens b) kann unter normalen, erhöhtem oder vermindertem Druck durchgeführt werden.

Bei der Durchführung des Verfahrens b) können beispielsweise etwa 1 bis 1,2 mol Tetrahydrophthalsäureanhydrid auf 1 mol der Verbindungen der Formel (IV) eingesetzt werden, und diese Verbindungen werden miteinander in Gegenwart von Essigsäure umgesetzt, so daß die angestrebten Verbindungen der Formel (I) erhalten werden können.

Bei der Durchführung des Verfahrens c) umfassen geeignete Verdünnungsmittel die gleichen Lösungsmittel, die beispielhaft für das Verfahren a) genannt sind.

Die Reaktionstemperaturen des Verfahrens c) können innerhalb eines Bereichs von beträchtlicher Breite variiert werden. Im allgemeinen wird die Reaktion bei einer Temperatur zwischen etwa -10 °C und etwa 100 °C, vorzugsweise zwischen etwa 10 °C und etwa 100 °C, durchgeführt.

Die Reaktion des Verfahrens c) kann unter normalem, erhöhtem oder vermindertem Druck durchgeführt werden.

Bei der Durchführung des Verfahrens c) können beispielsweise etwa 1 bis 1,2 mol der Verbindungen der Formel (VI) auf 1 mol der Verbindungen der Formel (V) eingesetzt werden, und diese Verbindungen werden miteinander in Gegenwart eines inerten Lösungsmittels umgesetzt und die dabei erhaltenen Produkte dann in Gegenwart einer Base cyclisiert, so daß die angestrebten Verbindungen der Formel (I) erhalten werden können.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotylendonen-Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen: Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte Natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohle wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-

Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in dem Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen etwa 0,0005 und etwa 3 kg/ha, vorzugsweise zwischen etwa 0,001 und etwa 2 kg/ha.

Die Herstellung und Verwendung der erfindungsgemäßen Verbindungen sind den folgenden Beispielen zu entnehmen.

<u>Herstellungsbeispiele</u>

<u>Beispiel 1</u>

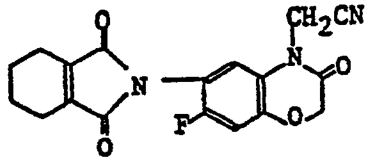

(Verbindung Nr. 1)

Eine Suspension von 2-[7-Fluoro-2H-1,4-benzoxazin-3(4H)-on-6-yl]-4,5,6,7-tetrahydro-2H-isoindol-1,3-dion (2g) und Kaliumcarbonat (1,05 g) in Acetonitril (30 ml) wird 30 min unter Rückfluß erhitzt. Die resultierende Lösung wird auf 5 °C abgekühlt. Der Lösung wird tropfenweise Chloroacetonitril (0,72 g) zugesetzt, und danach wird die Lösung 3 h unter Rückfluß erhitzt. Das Lösungsmittel wird abdestilliert, und der Rückstand wird mit Wasser vermischt und einem Arbeitsgang der Extraktion mit Dichloromethan unterworfen. Die Dichloromethan-Schicht wird abgetrennt, mit Wasser gewaschen und über Natriumsulfat

getrocknet. Die Lösung wird konzentriert, um ein kristallines Produkt abzu scheiden, das dann aus einem aus Acetonitril und Wasser bestehenden Lösungsmittel umkristallisiert wird. Als Endprodukt wird 2-[7-Fluoro-4-cyanomethyl-2H-1,4-benzoxazin-3(4H)-on-6-yl]-4,5,6,7-tetr hydro-2H-isoindol-1,3-dion (0,84 g) erhalten; Schmp. 216-220 °C.

Beispiel 2

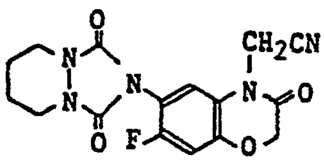

(Verbindung Nr. 2)

Eine Mischung aus 2-[7-Fluoro-2H-1,4-benzoxazin-3(4H)-on-6-yl]-hexahydro-1H-(1,2,4)-triazolo(1,2-a)-pyridazin-1,3-dion (2,04 g), Kaliumcarbonat (1,06 g), Chloroacetonitril (0,96 g) und Acetonitril (30 ml) wird 3 h unter Rückfluß erhitzt. Die Reaktionsmischung wird durch Eindampfen im Vakuum von Acetonitril befreit und mit Wasser vermischt. Der resultierende feste Stoff wird durch Filtration gesammelt und mit einer kleinen Menge Ethanol gewaschen, wonach als Endprodukt 2-[4-Cyanomethyl-7-fluoro-2H-1,4-benzoxazin-3(4H)-on-6-yl]-hexahydro-1H (1,2,4)-triazolo(1,2-a)-pyridazin-1,3-dion (1,62 g) erhalten wird; Schmp. 286-287 °C.

Beispiel 3

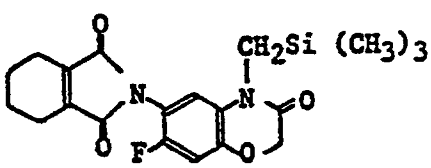

(Verbindung Nr. 3)

Zu N,N-Dimethylformamid (10 ml) werden bei Umgebungstemperatur Natriumhydrid (0,1 g), Natriumiodid (5 mg), 18-Krone-6 (5 mg) und 2-[7-Fluoro-2H-1,4-benzoxazin-3(4H)-on-6-yl]-4,5,6,7-tetrahydro-2H-isoindol-1,3-dion (0,63 g) hinzugefügt. Danach wird das Ganze 15 min gerührt, mit einer Lösung von Chloromethyltrimethylsilan (0, 61 g) in N,N-Dimethylformamid (5 ml) behandelt und 1 h bei 100-110 °C gerührt. Die Reaktionsmischung wird im Vakuum zur Trockne eingedampft und der Rückstand wird mit Wasser (30 ml) vermischt und zweimal mit Dichloromethan (30 ml) extrahiert. Der vereinigte Extrakt wird über Natriumsulfat getrocknet und im Vakuum eingedampft, wonach als öliges Endprodukt 2-[7-Fluoro-4-trimethylsilylmethyl-2H-1,4-benzoxazin-3(4H)-on-6-yl]-4,5,6,7-tetrahydro-2H-isoindol-1,3-dion (0,7 g) erhalten wurden; $n_D^{20}$ 1,5596.

Die Verbindungen der Formel (I) gemäß der vorliegenden Erfindung, die in der gleichen Weise wie in den Beispielen 1 bis 3 hergestellt werden können, sind in der nachstehenden Tabelle 1 aufgeführt.

Tabelle 1

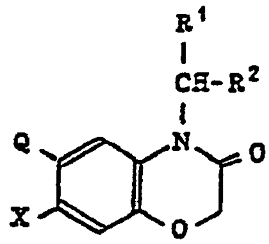

| Verb. Nr. | Q | X | $R^1$ | $R^2$ | |
|---|---|---|---|---|---|
| 4 | 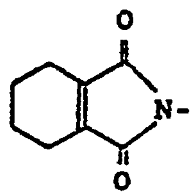 | H | $CH_3$ | -CN | Schmp. 189~190℃ |
| 5 | 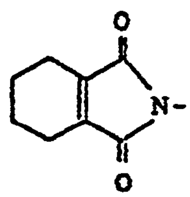 | F | $CH_3$ | -CN | Schmp. 159~160.5℃ |
| 6 | 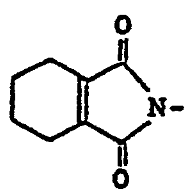 | F | H | $-SCH_3$ | |
| 7 | 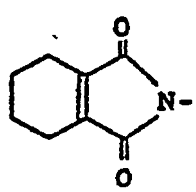 | F | H | $-SC_2H_5$ | |
| 8 | 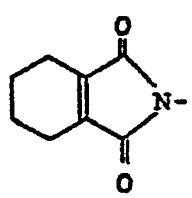 | H | H | $-Si(CH_3)_3$ | Viskoses Öl |

| Verb. Nr. | Q | X | $R^1$ | $R^2$ | |
|---|---|---|---|---|---|
| 9 | 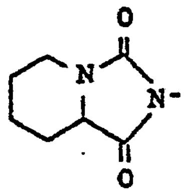 | H | H | -CN | Schmp. 209~213℃ |
| 10 | 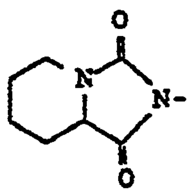 | F | H | -CN | Schmp. 269~271℃ |
| 11 | 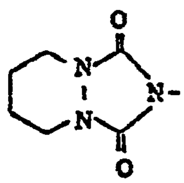 | H | H | -CN | Schmp. 247~249℃ |
| 12 | 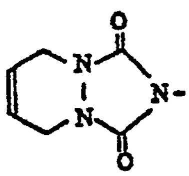 | H | H | -CN | Schmp. 268~272℃ |
| 13 | 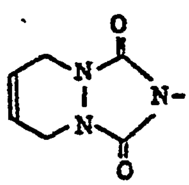 | F | H | -CN | Schmp. 233~237℃ |
| 14 | 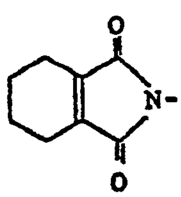 | F | H | 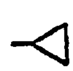 | Schmp. 133~134℃ |

| Verb. Nr. | Q | X | R[1] | R[2] | |
|---|---|---|---|---|---|
| 15 | 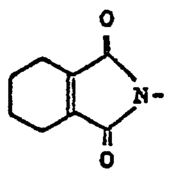 | H | H | -CN | Schmp. 180~182℃ |
| 16 | 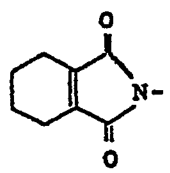 | H | H | $-\overset{O}{\overset{\|}{C}}OCH_2Si(CH_3)_3$ | $n_D^{50}$ 1.5516 |
| 17 | 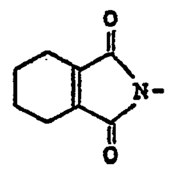 | F | H | $-\overset{O}{\overset{\|}{C}}OCH_2Si(CH_3)_3$ | $n_D^{20}$ 1.5446 |

Beispiel 4

(Herstellung eines Zwischenprodukts)

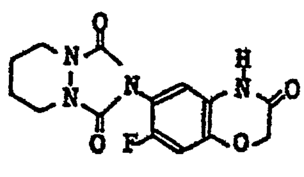

(Zwischenprodukt Nr. 1)

Eine aus 6-Isocyanato-7-fluoro-2H-1,4-benzoxazin-3(4H)-on (2,14 g), 1-Ethoxycarbonylhexahydropyridazin (1,62 g) und Dioxan (30 ml) bestehende Mischung wird mit einigen Tropfen Triethylamin behandelt, 2 h bei Umgebungstemperatur gerührt und über Nacht stehen gelassen. Die Mischung wird durch Abdampfen im Vakuum von dioxan befreit, mit Methanol (30 ml), das eine katalytische Menge Natrium enthält, vermischt und 2 h unter Rückfluß erhitzt. Nach der Entfernung des Methanols durch Abdampfen im Vakuum wird der resultierende feste Stoffe gesammelt und mit Wasser gewaschen, wonach als Endprodukt 2-[7-Fluoro-2H-1,4-benzoxazin3(4H)-on-6-yl]-hexahydro-1H-(1,2,4)-triazo o (1,2-a)-pyridazin-1,3-dion (2,45 g) erhalten wird; Schmp. 287-290 °C.

Einer der Ausgangsstoffe im vorstehenden Beispiel 4, 6-Isocyanato-7-fluoro-2H-1,4-benzoxazin-3(4H)-on, wird dadurch erhalten, daß 6-Amino-7-fluoro-2H-1,4-benzoxazin-3(4H)-on mit einer äquimolaren Menge oder einem kleinen Überschuß Trichloromethylchlorameisensäureester in Gegenwart von Dioxan während einer Zeitspanne zum Rückfluß erhitzt wird, die erforderlich ist, um aus der zum Rückfluß erhitzten Mischung eine klare Lösung entstehen zu lassen, die im vorstehenden Beispiel 4 nach der Entfernung der leicht flüchtigen Substanzen durch Abdampfen im Vakuum ohne Isolierung und Identifizierung eingesetzt werden kann.

Beispiel 5

(Herstellung eines Zwischenprodukts)

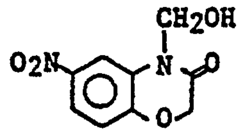

(Zwischenprodukt Nr. 2)

Eine aus 6-Nitro-2H-1,4-benzoxazin-3(4H)-on (5,82 g), einer 37-proz. wäßrigen Formaldehyd-Lösung (28,5 ml) und Wasser (5,4 ml) gerührte Lösung wird 2 h unter Rückfluß erhitzt und danach gekühlt. Der resultierende feste Stoff wird durch Filtration gesammelt, nacheinander mit Wasser, Ethanol, Ether und n-hexan gewaschen und getrocknet, wonach 4-Hydroxymethyl-6-nitro-2H-1,4-benzoxazin-3(4H)-on (5,19g) erhalten wurde; Schmp. 239 °C.

Die folgende Verbindung wird nach einem Verfahren ähnlich demjenigen des vorstehenden Beispiels 5 erhalten.

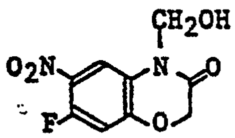

(Zwischenprodukt Nr. 3)

Beispiel 6

(Herstellung eines Zwischenprodukts)

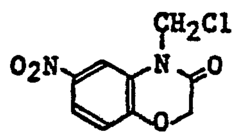

(Zwischenprodukt Nr. 4)

Zu einer Suspension von 4-Hydroxymethyl-6-nitro-2H-1,4-benzoxazin-3(4H)-on (22,42 g) in Chloroform (50 ml) wird eine katalytische Menge Triethylamin hinzugefügt. Die Mischung wird bei Umgebungstemperatur gerührt, tropfenweise mit Thionylchlorid behandelt und 6 h bei 60 °C gerührt. Die Reaktionsmischung wird durch Filtration von unlöslichen Stoffen befreit, und das Filtrat wird in Wasser gegossen. Die abgetrennte Chloroform-Schicht wird nacheinander mit Wasser, gesättigter Natriumhydrogencarbonat-Lösung und gesättigter Natriumchlorid-Lösung gewaschen, getrocknet und eingedampft, wonach 4-Chloromethyl-6-nitro-2H-1,4-benzoxazin-3(4H)-on (17,23 g) erhalten wird; Schmp. 133 °C.

Die folgende Verbindung wird nach einem Verfahren ähnlich demjenigen des vorstehenden Beispiels 6 erhalten.

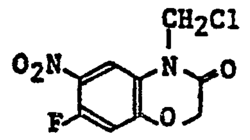

(Zwischenprodukt Nr. 5)

Beispiel 7

(Herstellung eines Zwischenprodukts)

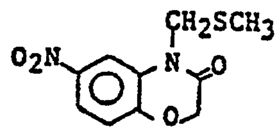

(Zwischenprodukt Nr. 6)

Methylmercaptan (1,9 g) wird langsam in eine zum Rückfluß erhitzte, aus 4-Chloromethyl-6-nitro-2H-1,4-benzoxazin-3(4H)-on (4,48 g), Kaliumcarbonat (3,3 g) und Methylethylketon (30 ml) bestehende Mischung eingeleitet. Die Reaktionsmischung wird 5 h unter Rückfluß erhitzt, von Methylethylketon durch Abdampfen im Vakuum befreit, und der resultierende Rückstand wird mit Ethylacetat extrahiert. Der Extrakt wird mit gesättigter wäßriger Natriumcarbonat-Lösung und mit Wasser gewaschen, über Natriumsulfat getrocknet und kondensiert, um eine Kristallisation sicherzustellen. Der erhaltene feste Stoff wird mit einer kleinen Menge Ether gewaschen, wonach 4-Methylthiomethyl-6-nitro-2H-1,4-benzoxazin-3 (4H)-on (2,3 g) erhalten wird; Schmp. 147 °C.

Beispiel 8

(Herstellung eines Zwischenprodukts)

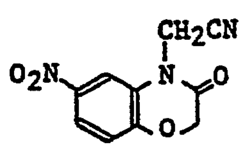

(Zwischenprodukt Nr. 7)

Rauchende (Dichte 1,52 g/cm³) Salpetersäure (1,3 ml) wird tropfenweise bei 0 °C zu Acetanhydrid (11,3 g) hinzugefügt, und anschließend wird eine katalytische Menge konz. Schwefelsäure zugesetzt. Zu der Lösung wird bei 0 °C portionsweise 4-Cyanomethyl-2H-1,4-benzoxazin-3(4H)-on (1,88 g) hinzugefügt. Die Mischung wird 20 min bei 0°C gerührt, tropfenweise mit einer kalten Lösung, die durch Auflösen von Natriumhydroxid (10 g) in Wasser (87,5 ml) und Kühlen auf 0 °C hergestellt wurde, behandelt, zur Beendigung der Reaktion 30 min bei 0 °C gerührt und mit Methylisobutylketon extrahiert. Der Extrakt wird mit Wasser gewaschen, getrocknet und eingedampft. Der erhaltene feste Stoffe wird aus Ethanol umkristallisiert, wonach als Endprodukt 4-Cyanomethyl-6-nitro-2H-1,4-benzoxazin-3(4H)-on (1,3 g) erhalten wird; Schmp. 173 °C.

Beispiel 9

(Herstellung eines Zwischenprodukts)

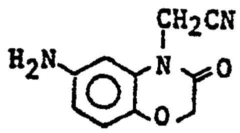

(Zwischenprodukt Nr. 8)

Eine Mischung aus Ethanol (15 ml), Essigsäure (1 g), Wasser (25 ml) und Eisen (3 g) wird bei 80 °C gerührt, und 4-Cyanomethyl-6-nitro-2H-1,4-benzoxazin-3(4H)-on (2,33 g) wird portionsweise dazugegeben. Das Ganze wird bei 80 °C gerührt, bis die Gasentwicklung aufhört. Die Reaktionsmischung wird mit Aktivkohle (1 g) und Ethanol (20 ml) vermischt, 15 min zum Rückfluß erhitzt und filtriert. Das Filtrat wird in gekühltes Wasser gegossen. Die abgeschiedenen Kristalle werden durch Filtration gesammelt und getrocknet, wonach 6-Amino-4-cyanomethyl-2H-1, 4-benzoxazin-3(4H)-on (1,51 g) erhalten wird; Schmp. 158 °C.

Zwischenprodukte, die in der gleichen Weise wie in den Beispielen 5 bis 9 hergestellt werden können, sind in der folgenden Tabelle 2 aufgeführt.

Tabelle 2

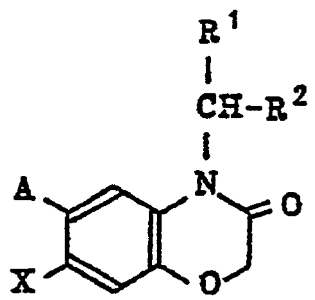

| Zwischenprodukt Nr. | A | X | $R^1$ | $R^2$ | |
|---|---|---|---|---|---|
| 9 | H | H | H | CN | Schmp. 115°C |
| 10 | H | F | H | CN | Schmp. 111°C |
| 11 | H | Cl | H | CN | Schmp. 132°C |
| 12 | $O_2N$ | F | H | CN | Schmp. 205°C |
| 13 | $O_2N$ | F | $CH_3$ | CN | |
| 14 | $O_2N$ | Cl | H | CN | Schmp. 187°C |
| 15 | $O_2N$ | H | H | $SC_2H_5$ | |
| 16 | $H_2N$ | F | H | CN | Schmp. 167°C |
| 17 | $H_2N$ | Cl | H | CN | Schmp. 180°C |
| 18 | $H_2N$ | H | $CH_3$ | CN | |
| 19 | $H_2N$ | F | $CH_3$ | CN | |
| 20 | $H_2N$ | H | H | $SCH_3$ | |
| 21 | $H_2N$ | H | H | $SC_2H_5$ | |
| 22 | $H_2N$ | F | H | $SCH_3$ | |
| 23 | $H_2N$ | F | H | $SC_2H_5$ | |

Biologische Tests

Vergleichs-Verbindung (E-1)

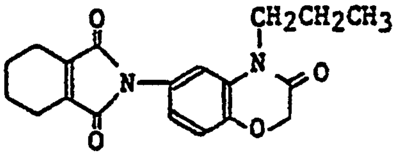

(die in der JP-OS 30586/1986 beschriebene Verbindung)

Beispiel 10

Test gegen Unkräuter in einem überfluteten Reisfeld durch Wasseroberflächen-Anwendung

Herstellung eines Wirkstoff-Präparats

Träger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegt Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie einjährigen breitblättrigen Unkräutern [Falsche Pimpernelle (Lindernia pyxidaria L.), Rotala indica, Amerikanischer Wasserwurz (Elatine triandra), Rotstengel (Ammannia multiflora Roxburgh) und Dopatrium junceum Hamilton] wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats, mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizität gegenüber Reis (bewertet unter Bezug auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind anhand typischer Beispiele in Tabelle 3 aufgeführt.

Tabelle 3

| Verbindung Nr. | Wirkstoff-Menge kg/ha | Herbizide Wirkung: Hühnerhirse | Herbizide Wirkung: Schirmkraut | Herbizide Wirkung: Monochoria | Herbizide Wirkung: Breitblättrige Unkräuter | Phytotoxizität gegen Reis |
|---|---|---|---|---|---|---|
| 5 | 0,25 | 5 | 5 | 5 | 5 | 2 |
| | 0,125 | 4 | 5 | 5 | 5 | 1 |
| | 0,06 | 4 | 5 | 5 | 5 | 0 |
| 15 | 0,25 | 5 | 5 | 5 | 5 | 2 |
| | 0,125 | 4 | 5 | 5 | 5 | 1 |
| | 0,06 | 4 | 5 | 5 | 5 | 0 |
| Vergleichs-Verbindung | | | | | | |
| E-1 | 0,5 | 5 | 5 | 5 | 5 | 4 |
| | 0,25 | 3 | 5 | 4 | 4 | 3 |
| | 0,125 | 1 | 4 | 2 | 2 | 2 |

Beispiel 11

Test gegen Unkräuter auf höher gelegenem Ackerboden durch Bodenbehandlung vor dem Auflaufen:

In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 10, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 10 geprüft. Die Ergebnisse sind anhand typischer Beispiele in Tabelle 4 aufgeführt.

Tabelle 4

| Verbindung Nr. | Wirkstoff-Menge kg/ha | Herbizide Wirkung Hühnerhirse | Grauer Amaranth | Gänsefuß | Phytotoxizität gegen Sojabohnen |
|---|---|---|---|---|---|
| 1 | 0,25 | 5 | 5 | 5 | 1 |
| | 0,125 | 4 | 5 | 5 | 0 |
| | 0,06 | 3 | 5 | 5 | 0 |
| 5 | 0,25 | 5 | 5 | 5 | 1 |
| | 0,125 | 4 | 5 | 5 | 0 |
| | 0,06 | 3 | 5 | 5 | 0 |
| Vergleichs-Verbindung | | | | | |
| E-1 | 0,5 | 4 | 5 | 5 | 4 |
| | 0,25 | 3 | 5 | 5 | 3 |
| | 0,125 | 3 | 4 | 3 | 3 |

Beispiel 12

Test gegen Unkräuter auf höher gelegenem Ackerboden durch Laub-Behandlung:

In einem Gewächshaus wurden Weizen-Samen in Töpfe von 500 cm² Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 10, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 10 geprüft. Die Ergebnisse sind anhand typischer Beispiele in Tabelle 5 aufgeführt.

**Tabelle 5**

| Ver-bin-dung Nr. | Wirkstoff-Menge kg/ha | Herbizide Wirkung Digi-taria | Herbizide Wirkung Grauer Amaranth | Herbizide Wirkung Gänse-fuß | Phytotoxi-zität gegen Weizen |
|---|---|---|---|---|---|
| 2 | 0,25 | 5 | 5 | 5 | 1 |
| | 0,125 | 5 | 5 | 5 | 0 |
| | 0,06 | 4 | 5 | 5 | 0 |
| 10 | 0,5 | 5 | 5 | 5 | 1 |
| | 0,25 | 5 | 5 | 5 | 0 |
| | 0,125 | 4 | 5 | 5 | 0 |
| 16 | 1 | 5 | 5 | 5 | 1 |
| | 0,5 | 5 | 5 | 5 | 0 |
| | 0,25 | 4 | 5 | 5 | 0 |
| 17 | 0,5 | 5 | 5 | 5 | 1 |
| | 0,25 | 5 | 5 | 5 | 0 |
| | 0,125 | 4 | 5 | 5 | 0 |
| Vergleichs-Verbindung | | | | | |
| E-1 | 1 | 5 | 5 | 5 | 4 |
| | 0,5 | 5 | 5 | 5 | 3 |
| | 0,25 | 4 | 5 | 5 | 3 |

## Patentansprüche

1. Neue Benzoxazine der Formel (I)

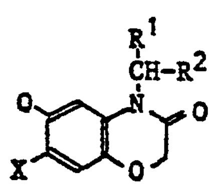

(I)

in der

X Wasserstoff oder Halogen ist,
$R^1$ Wasserstoff oder $C_1$-$C_2$-Alkyl ist,
$R^2$Cyano, Trimethylsilyl, Trimethylsilylmethoxycarbonyl, $C_1$-$C_4$-Alkylthio oder Cyclopropyl ist und
Q eine der folgenden Gruppen ist

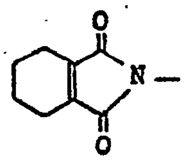 , 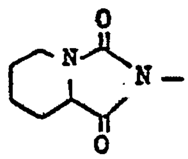 , 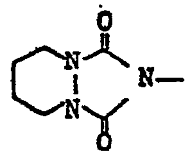 oder 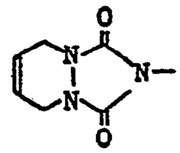

2. Verbindungen der Formel (I) nach Anspruch 1, dadurch gekennzeichnet, daß
X Wasserstoff, Fluor oder Chlor ist,
$R^1$ Wasserstoff oder Methyl ist,
$R^2$ Cyano, Trimethylsilyl, Trimethylsilylmethoxycarbonyl, Methylthio oder Cyclopropyl ist und
Q eine der folgenden Gruppen ist

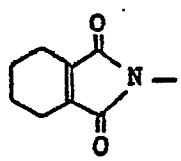 , 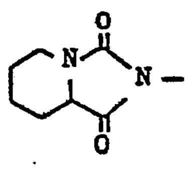 , 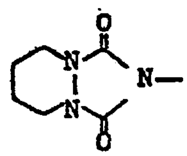 oder 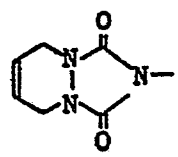

3. Verbindung der Formel (I) nach Anspruch 1, dadurch gekennzeichnet, daß
X Wasserstoff oder Fluor ist,
$R^1$ Wasserstoff oder Methyl ist,
$R^2$ Cyano ist und
Q eine der folgenden Gruppen ist

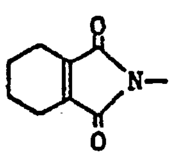 , 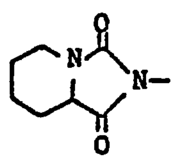 , 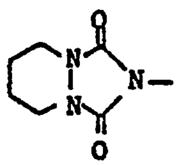 oder 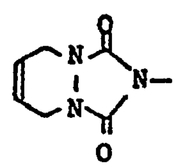

4. Verbindung nach Anspruch 1:
2-[4-Cyanomethyl-7-fluoro-2H-1,4-benzoxazin-3(4H)-on-6-yl]-4,5,6,7-tetrahydro-2H-isoindol-1,3-dion der Formel

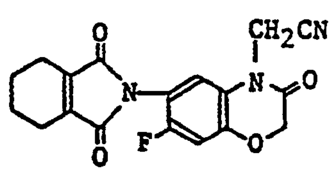

2-[4-(1-Cyanoethyl)-7-fluoro-2H-1,4-benzoxazin-3(4H)-on-6-yl]-4,5,6,7-tetrahydro-2H-isoindol-1,3-dion der Formel

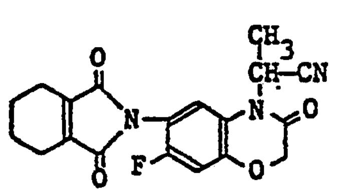

3-[4-Cyanomethyl-7-fluoro-2H-1,4-benzoxazin-3(4H)-on-6-yl]1,5-tetramethylen-1,3-diazolidin-2,4-di-

on der Formel

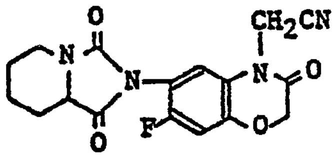

und

2-[4-Cyanomethyl-7-fluoro-2H-1,4-benzoxazin-3(4H)-on-6-yl]-hexahydro-1H-(1,2,4)triazolo(1,2-a)pyridazin-1,3-dion der Formel

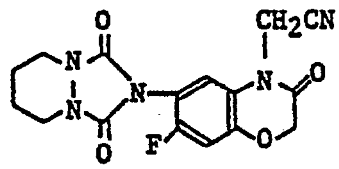

5. Verfahren zur Herstellung neuer Benzoxazine der Formel (I)

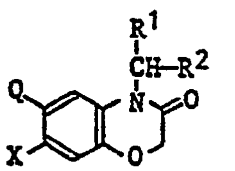

(I),

in der
X Wasserstoff oder Halogen ist,
$R^1$ Wasserstoff oder $C_1$-$C_2$-Alkyl ist,
$R^2$ Cyano, Trimethylsilyl, Trimethylsilylmethoxycarbonyl, $C_1$-$C_4$-Alkylthio oder Cyclopropyl ist und
Q eine der folgenden Gruppen ist

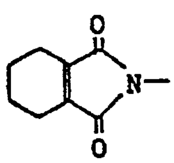 , 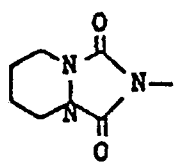 , 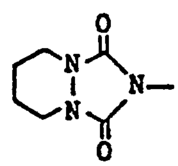 oder 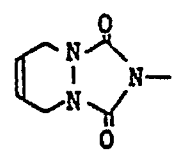

dadurch gekennzeichnet, daß
a) Verbindungen der Formel (II)

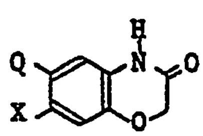

(II)

in der X und Q die oben angegebenen Bedeutungen haben, mit Verbindungen der Formel (III)

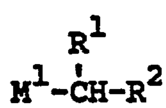

(III).

in der $R^1$ und $R^2$ die oben angegebenen Bedeutungen haben und $M^1$ Halogen ist, in Gegenwart eines inerten Lösungsmittels, gegebenenfalls in Gegenwart einer Base, umgesetzt werden
oder
b) in dem Fall, in dem Q die folgende Gruppe ist

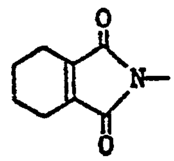

Verbindungen der Formel (IV)

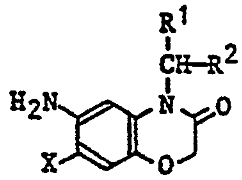

(IV)

in der X, $R^1$ und $R^2$ die oben angegebenen Bedeutungen haben, mit Tetrahydrophthalsäureanhydrid der Formel

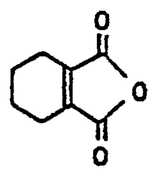

in Gegenwart von Essigsäure umgesetzt werden oder

c) in dem Fall, in dem Q eine der folgenden Gruppen ist

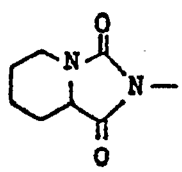 , 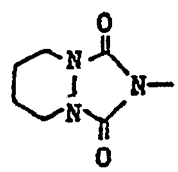 oder 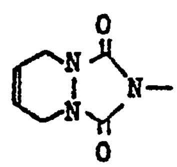

Verbindungen der Formel (V)

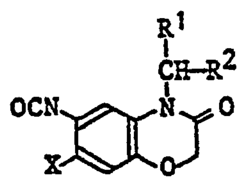

(V)

in der X, $R^1$ und $R^2$ die oben angegebenen Bedeutungen haben, mit Verbindungen der Formel (VI)

W - C O O T(VI),

in der W eine der folgenden Gruppen ist

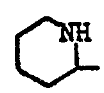

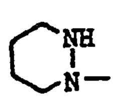 oder 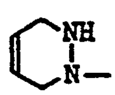

und

T Wasserstoff oder Alkyl ist,

in Gegenwart eines inerten Lösungsmittels umgesetzt werden und dann die genannten Produkte in Gegenwart einer Säure einer Base cyclisiert werden.

6. Herbizide Mittel, dadurch gekennzeichnet, daß sie wenigstens ein Benzoxazin der Formel (I) nach den Ansprüchen 1 bis 4 enthalten.

7. Verfahren zur Bekämpfung von Unkräutern, dadurch gekennzeichnet, daß Benzoxazine der Formel (I) nach den Ansprüchen 1 bis 4 auf Unkräuter und/oder deren Lebensraum zur Einwirkung gebracht werden.

8. Verwendung von Benzoxaxinen der Formel (I) nach den Ansprüchen 1 bis 4 zur Bekämpfung von Unkräutern.

9. Verfahren zur Herstellung herbizider Mittel, dadurch gekennzeichnet, daß Benzoxazine der Formel (I) nach den Ansprüchen 1 bis 4 mit Streckmitteln und/oder grenzflächenaktiven Mitteln vermischt werden.

10. Verbindungen der Formel (XIX)

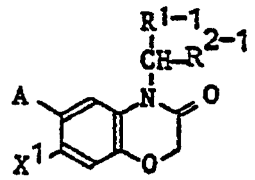

(XIX)

in der

$R^{1-1}$ Wasserstoff, Methyl oder Ethyl ist,

$R^{2-1}$ Cyano, Hydroxy, Chlor, Trimethylsilyl, Trimethylsilylmethoxycarbonyl, $C_1$-$C_4$-Alkylthio oder Cyclopropyl ist,

A Wasserstoff, Amino oder Nitro ist und

$X^1$ Wasserstoff oder Halogen ist,

mit der Maßgabe, daß, wenn A Nitro ist, $R^{2-1}$ Chlor oder Hydroxy ist.

## Claims

1. Novel benzoxazines of formula (I)

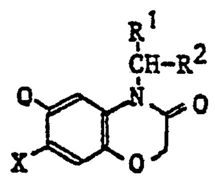

(I)

in which

X is hydrogen or halogen,

$R^1$ is hydrogen or $C_1$–$C_2$-alkyl,

$R^2$ is cyano, trimethylsilyl, trimethylsilylmethoxycarbonyl, $C_1$–$C_4$-alkylthio or cyclopropyl and

Q is one of the groups which follow

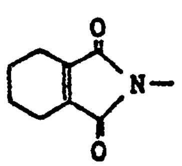 , 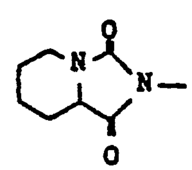 , 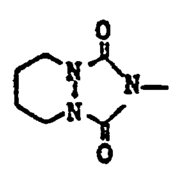 or 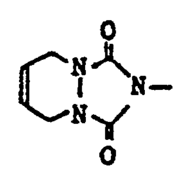

2. Compounds of the formula (I) according to claim 1, characterized in that

X is hydrogen, fluorine or chlorine,

$R^1$ is hydrogen or methyl,

$R^2$ is cyano, trimethylsilyl, trimethylsilylmethoxycarbonyl, methylthio or cyclopropyl and

Q is one of the groups which follow

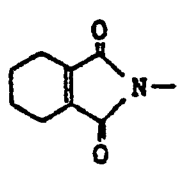 , 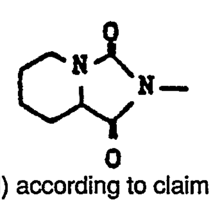 , 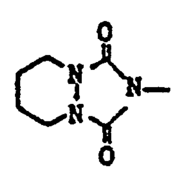 or 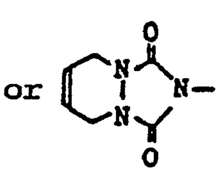

3. Compounds of the formula (I) according to claim 1, characterized in that

X is hydrogen or fluorine,
$R^1$ is hydrogen or methyl,
$R^2$ is cyano and
Q is one of the groups which follow

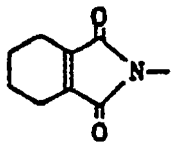 , 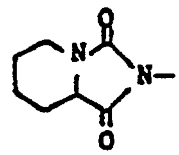 , 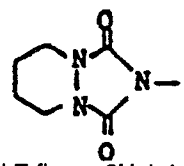 or 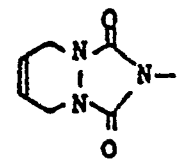

4. Compounds according to claim 1: 2-[4-cyanomethyl-7-fluoro-2H-1,4-benzoxazin-3(4H)-on-6yl]-4,5,6,7-tetrahydro-2H-isoindole-1,3-dione of the formula

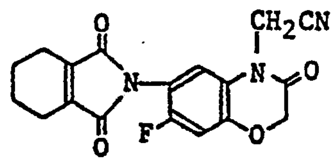

2-[4-(1-cyanoethyl)-7-fluoro-2H-1,4-benzoaxazin-3(4H)-on-6-yl]-4,5,6,7-tetrahydro-2H-isoindole-1,3-dione of the formula

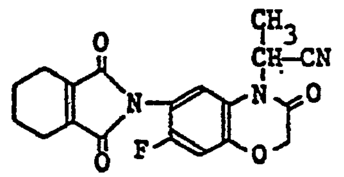

3-[4-cyanomethyl-7-fluoro-2H-1,4-benzoxazin-3(4H)-on-6-yl]-1,5-tetramethylene-1,3-diazolidine-2,4-dione of the formula

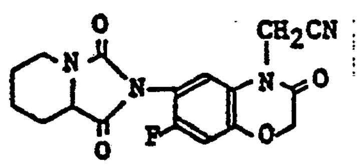

and
2-[4-cyanomethyl-7-fluoro-2H-1,4-benzoaxzin-3(4H)-on-6yl]-hexahydro-1H-(1,2,4)triazolo(1,2-a)pyridazine-1,3-dione of the formula

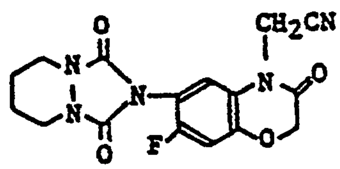

5. Process for the preparation of novel benzoxazines of the formula (I)

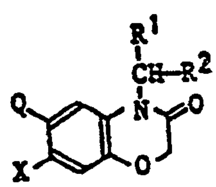

(I),

in which
X is hydrogen or halogen,
$R^1$ is hydrogen or $C_1$–$C_2$-alkyl,
$R^2$ is cyano, trimethylsilyl, trimethylsilylmethoxycarbonyl, $C_1$–$C_4$-alkylthio or cyclopropyl and
Q is one of the groups which follow

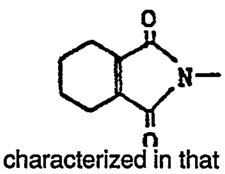 , 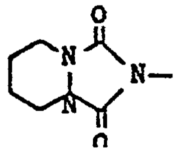 , 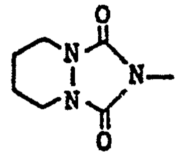 oder 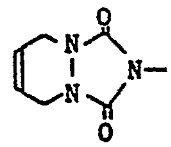

characterized in that

a) compounds of the formula (II)

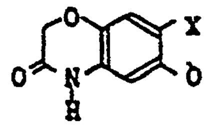

(II)

in which X and Q have the meanings given above are reacted with compounds of the formula (III)

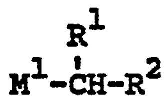

(III).

in which $R^1$ and $R^2$ have the meanings given above and $M^1$ is halogen, in the presence of an inert solvent and optionally in the presence of a base, or

b) if Q is the group which follows

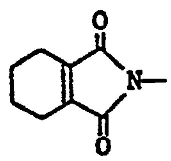

compounds of the formula (IV)

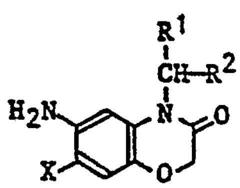

(IV)

in which X, $R^1$ and $R^2$ have the meanings given above, are reacted with tetrahydrophthalic anhydride of the formula

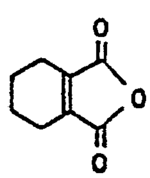

in the presence of acetic acid or

c) if Q is one of the groups which follow

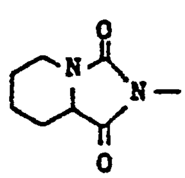 , 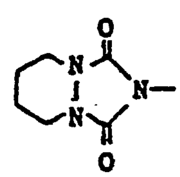 or 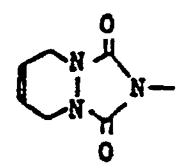

compounds of the formula (V)

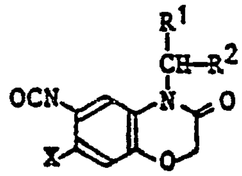

(V)

in which X, $R^1$ and $R^2$ have the meanings given above, are reacted with compounds of the formula (VI)

W–COOT

in which W is one of the groups which follow

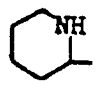

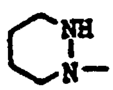

or

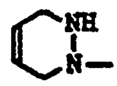

and

and T is hydrogen or alkyl, in the presence of an inert solvent, and then the abovementioned products are cyclized in the presence of an acid or a base.

6. Herbicidal agents, characterized in that they contain at least one benzoxazine of the formula (I) according to claims 1 to 4.

7. Method for combating weeds, characterized in that benzoxazines of the formula (I) according to claims 1 to 4 are allowed to take effect on weeds and/or the habitat thereof.

8. The use of benzoxazines of the formula (I) according to Claims 1 to 4 for combating weeds.

9. Process for the preparation of herbicidal agents, characterized in that benzoxazines of the formula (I) according to claims 1 to 4 are mixed with extenders and/or surface-active agents.

10. Compounds of the formula (XIX)

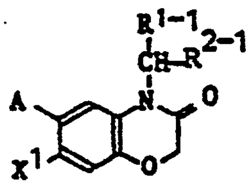

(XIX)

in which

$R^{1-1}$ is hydrogen, methyl or ethyl,

$R^{2-1}$ is cyano, hydroxyl, chlorine, trimethylsilyl, trimethylsilylmethoxycarbonyl, $C_1$–$C_4$-alkylthio or cyclopropyl,

A is hydrogen, amino or nitro and

$X^1$ is hydrogen or halogen, with the proviso that if A is nitro, $R^{2-1}$ is chlorine or hydroxyl.

## Revendications

1. Nouvelles benzoxazines de formule I:

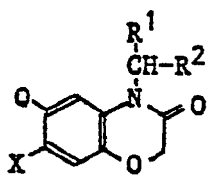

(I)

dans laquelle
X représente l'hydrogène ou un halogène,
$R^1$ représente l'hydrogène ou un groupe alkyle en $C_1$–$C_2$,
$R^2$ représente un groupe cyano, triméthylsilyle, triméthylsilylméthoxycarbonyle, alkylthio en $C_1$–$C_4$ ou cyclopropyle, et
Q représente l'un des groupes suivants:

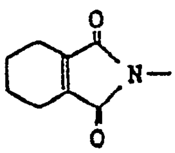 , 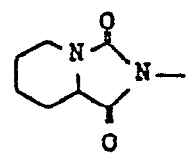 , 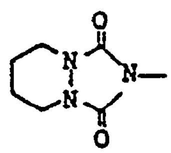 ou 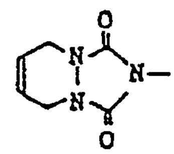

2. Composés de formule I selon la revendication 1, caractérisés en ce que:
X représente l'hydrogène, le fluor ou le chlore,
$R^1$ représente l'hydrogène ou un groupe méthyle,
$R^2$ représente un groupe cyano, triméthylsilyle, triméthylsilylméthoxycarbonyle, méthylthio ou cyclopropyle, et
Q représente l'un des groupe suivants:

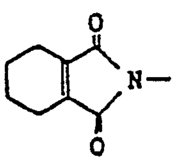 , 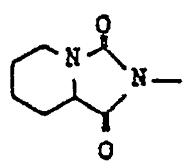 , 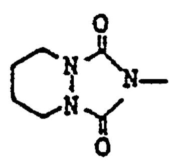 ou 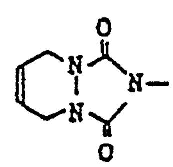

3. Composés de formule I selon la revendication 1, caractérisés en ce que:
X représente l'hydrogène ou le fluor,
$R^1$ représente l'hydrogène ou un groupe méthyle,
$R^2$ représente un groupe cyano, et
Q représente l'un des groupes suivants:

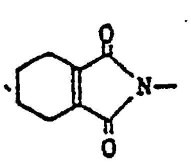 , 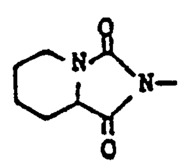 , 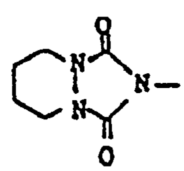 ou 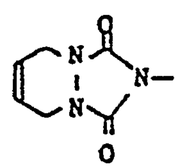

4. Composés selon la revendication 1: la 2-[4-cyanométhyl-7-fluoro-2H-1,4-benzoxazine-3(4H)-one-6-yl]-4,5,6,7-tétrahydro-2H-isoindole-1,3-dione de formule:

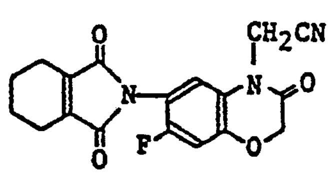

la 2-[4-(1-cyanéthyl)-7-fluoro-2H-1,4-benzoxazine-3(4H)-one-6-yl]-4,5,6,7-tétrahydro-2H-isoindole-1,3-dione de formule:

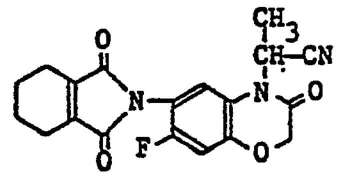

la 3-[4-cyanométhyl-7-fluoro-2H-1,4-benzoxazine-3(4H)-one-6-yl]-1,5-tétra-méthylène-1,3-diazolidi ne-2,4-dione de formule:

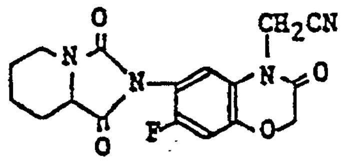 et

la 2-[4-cyanométhyl-7-fluoro-2H-1,4-benzoxazine-3(4H)-one-6-yl]-hexahydro-1H-(1,2,4)triazolo(1,2-a) pyridazine-1,3-dione de formule:

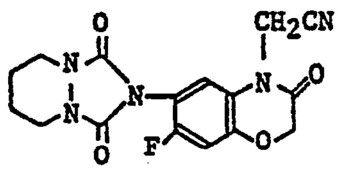

5. Procédé de préparation de nouvelles benzoxazines de formule I:

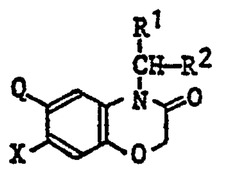

(I),

dans laquelle

X représente l'hydrogène ou un halogène,

$R^1$ représente l'hydrogène ou un groupe alkyle en $C_1$–$C_2$,

$R^2$ représente un groupe cyano, triméthylsilyle, triméthylsilylméthoxycarbonyle, alkylthio en $C_1$–$C_4$ ou cyclopropyle, et

Q représente l'un des groupes suivants:

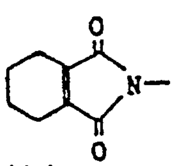 , 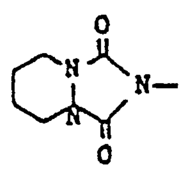 , 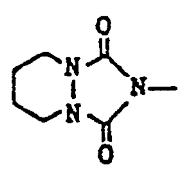 ou 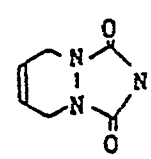

caractérisé en ce que:

a) on fait réagir des composés de formule II:

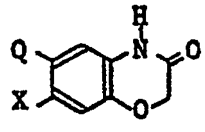

(II)

dans laquelle X et Q ont les significations indiquées ci-dessus, avec des composés de formule III:

$$\begin{array}{c} R^1 \\ | \\ M^1-CH-R^2 \end{array} \quad (III)$$

dans laquelle $R^1$ et $R^2$ ont les significations indiquées ci-dessus et $M^1$ représente un halogène, en présence d'un solvant inerte, eventuellement en présence d'une base ou bien

b) dans le cas où Q représente le groupe suivant:

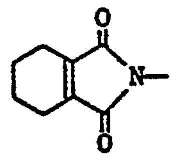

on fait réagir des composés de formule IV:

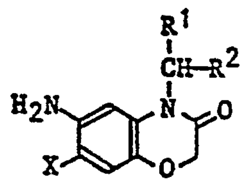

(IV)

dans laquelle X, $R^1$ et $R^2$ ont les significations indiquées ci-dessus, avec l'anhydride tétrahydrophtalique de formule:

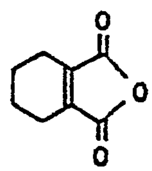

en présence d'acide acétique, ou bien

c) dans le cas où Q représente l'un des groupes suivants:

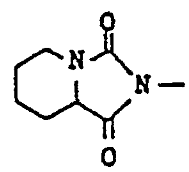 , 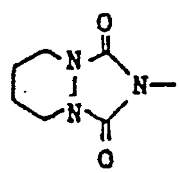 ou 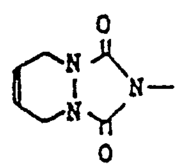

on fait réagir des composés de formule

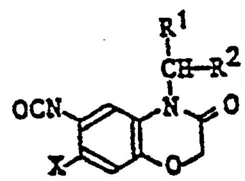

(V)

dans laquelle X, $R^1$ et $R^2$ ont les significations indiquées ci-dessus, avec des composés de formule VI:

W - C O O T (VI),

dans laquelle W représente l'un des groupes suivants:

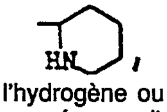 , 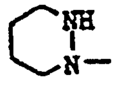 ou 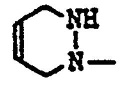 et

T représente l'hydrogène ou un groupe alkyle, en présence d'un solvant inerte puis on cyclise les produits obtenus en présence d'un acide ou d'une base.

6. Produits herbicides, caractérisés en ce qu'ils contiennent au moins une benzoxazine de formule I selon les revendications 1 à 4.

7. Procédé pour combattre les végétaux adventices, caractérisé en ce que l'on fait agir sur ces végétaux et/ou leur habitat des benzoxazines de formule I selon les revendications 1 à 4.

8. Utilisation des benzoxazines de formule I selon les revendications 1 à 4, pour la lutte contre les végétaux adventices.

9. Procédé de préparation de produits herbicides, caractérisé en ce que l'on mélange des benzoxazines de formule I selon les revendications 1 à 4 avec des diluants et/ou des agents tensioactifs.

10. Composés de formule XIX:

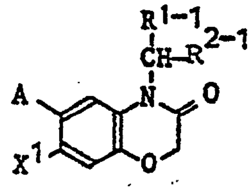

(XIX)

dans laquelle:

$R^{1-1}$ représente l'hydrogène, un groupe méthyle ou éthyle,

$R^{2-1}$ représente un groupe cyano, hydroxy, le chlore, un groupe triméthylsilyle, triméthylsilylméthoxycarbonyle, alkylthio en $C_1$–$C_4$ ou cyclopropyle,

A représente l'hydrogène, un groupe amino ou nitro, et

$X^1$ représente l'hydrogène ou un halogène, sous réserve que, lorsque A représente un groupe nitro, $R^{2-1}$ représente le chlore ou un groupe hydroxy.